# EUROPEAN PATENT SPECIFICATION

(11) **EP 1 023 918 B1**
(45) Date of publication and mention of the grant of the patent: **22.11.2006**
(21) Application number: 00101601.3
(22) Date of filing: 27.01.2000
(51) Int. Cl.: A61N 1/368, A61N 1/362

(54) **Pacemaker system with diurnal pattern controlled overdrive for prevention of tachycardia**
Herzschrittmachersystem mit durch das tägliche Muster gesteuerter Schnellgang zur Vorbeugung einer Tachykardie
Sytème de stimulation cardiaque avec surmultiplication commandée par le diagramme diurne pour la prévention d'une tachycardie

(30) Priority: 27.01.1999 US 237815
(43) Date of publication of application: 02.08.2000
(73) Proprietor: MEDTRONIC, INC., Minneapolis, Minnesota 55432-5604 (US)
(72) Inventor: Stoop, Gustaaf A.P., 6951 LS Dieren (NL); Van Oort, Geeske, 7711 JL Nieuwleusen (NL); Van Groeningen, Christianus J.J.E., 3512 En Utrecht (NL); De Vries, Bernhard A.P., 6951 AC Dieren (NL)
(74) Representative: Hughes, Andrea Michelle

(56) References cited:
- US-A- 5 042 497
- US-A- 5 560 368
- US-A- 5 861 011
- US-A- 5 967 995
- US-A- 6 161 041

## Description

This invention relates to cardiac pacemaker systems and, more particularly, cardiac pacemaker systems with techniques for anticipating tachycardia and the capacity to automatically switch to a pacing modality designed to prevent Ventricular Tachycardia.

The prevention of ventricular tachycardia and other dangerous arrhythmias has become a subject of intensive study and research. Ventricular tachycardia (VT) is a lethal arrhythmia which is known to frequently lead to sudden death, usually after progressing to ventricular fibrillation (VF). As a result, there have been many investigations into techniques for monitoring a patient's cardiac condition to determine when conditions indicate a likelihood of VT, and to otherwise continually assess the risk of VT and VF.

It is known that QT interval, and in particular abnormalities of the QT interval, are associated with ventricular arrhythmias, e.g., in long QT syndromes and after myocardia infarction. See "Diurnal Variation of the QT Interval - Influence of the Autonomic Nervous System," Bexton et al., British Heart J., 1986; 55:253-8. Thus, the QT interval of the surface EKG is accepted as an indirect measure of patient myocardial depolarization and repolarization. It is known that QT is longer during sleep, and in fact QT corrected for rate (QTc) is also longer during sleep. Further, it has been noted that QTc interval and QTc variability reach peak shortly after awakening hours, which may reflect increased automatic instability during early waking hours; and further that the time of the peak value corresponds to the period of reported increased vulnerability to ventricular tachycardia and sudden cardiac death. Molnar et al., J. Am. Coll. Cardiology, 1996 Jan., 27:1, 76-83. See also Christenson et al., PACE, Vol. 19, September 1996, 1296-1393, stating that QTc exhibits significant diurnal variability. The literature suggests that there are significant changes in the autonomic system during sleep, with either an increase in para-sympathetic tone, or an increase in sympathetic activity, or both. There is also a diurnal variation in circulating catecholamines, and specifically the catecholamine level drops during the night. These factors are known to influence repolarization, nighttime variations of which are reflected in the lengthening QT interval, and lengthened QTc. Other investigations have looked into the temporal and spatial distribution of QT intervals, and suggest that QT dispersion (QTd), measured as the difference between the maximal and minimal value of QT duration, can be associated with increased risk of ventricular tachycardia and sudden cardiac death. Accordingly, the literature presently suggests that both QT interval prolongation and increased QT dispersion (refractory dispersion), or dispersion of the repolarization duration, are important reflectors of the risk of an incipient ventricular tachycardia. A pacemaker system according to the preamble of claim 1 is known from document US 5 560 368.

This invention is responsive to the need to address the increased vulnerability to attacks of VT during or just after the waking period, and utilize the fact that the awakening period is characterized by observable variabilities of the depolarization and repolarization waves and the QT duration. In particular, the invention meets the need for a pacemaker which incorporates the capacity to monitor the diurnal heart pattern, particularly changes of the QRS-T waveform during the awakening period, so as to provide an indication of a risk of VT. A pacemaker in accordance with this invention also automatically provides for a controlled pacing response designed to prevent the onset of VT during the vulnerable awakening period.

It is a primary object of this invention to provide a pacemaker system having diurnal pattern detection for determining, on the basis of monitored QT interval, a period of patient awakening which covers the period when cardiac patients are most vulnerable to onset of VT; and to combine in the pacemaker system a continuous determination throughout the awakening period of data indicative of the likelihood of incipient VT, combined with a pacing response mode for preventing VT when it is determined to be likely.

In accordance with the above objects, there is provided a pacemaker system for pacing a patient's heart, having ventricular pacing (VP) means for generating and delivering pacing pulses to a patient's ventricle, rate control means for controlling the rate of said pacing pulses, and said rate control means having VT prevention means for controlling said rate to an intervention rate when said patient has a detected measure of refractory dispersion during awakening, said VT prevention means comprising:
awakening means for determining when said patient is in a state of awakening;
dispersion means for determining a measure of dispersion of ventricular refractoriness; and
intervention means for setting and continually adjusting said intervention rate as a function of said dispersion measure.

In accordance with a preferred embodiment, the dispersion means comprises
dispersion data means for determining during said awakening period data representative of ventricular refractory dispersion; and the system further comprises
threshold means for determining from said data when patient refractory dispersion exceeds said threshold; and the intervention means comprises overdrive control means for setting pacing rate at an intervention rate which is adjusted as a function of said data.

In accordance with another embodiment, the dispersion means comprises
depolarization means for determining a first measure of dispersion of the patient's depolarization wave during awakening;
repolarization means for determining a second measure of dispersion of the patient's repolarization wave during awakening.

Preferred embodiments of the invention will now be described, by way of example only, with reference to the accompanying drawings.
Figure 1 is a block diagram illustrating the primary functional components of a pacemaker system in accordance with this invention.

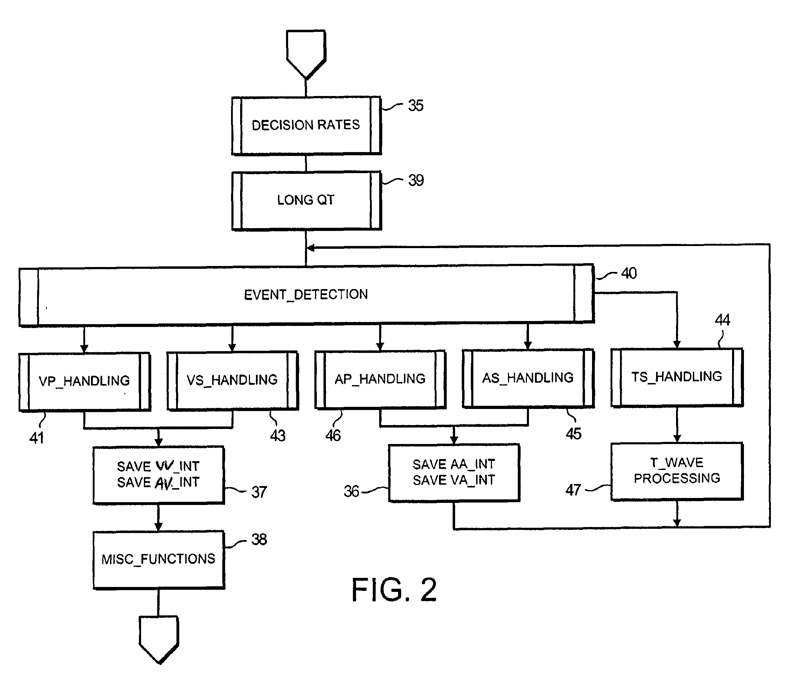

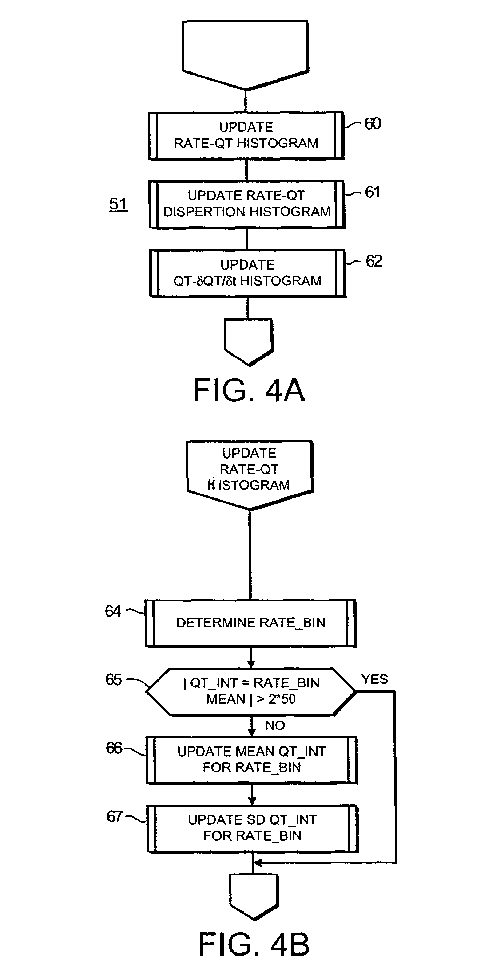

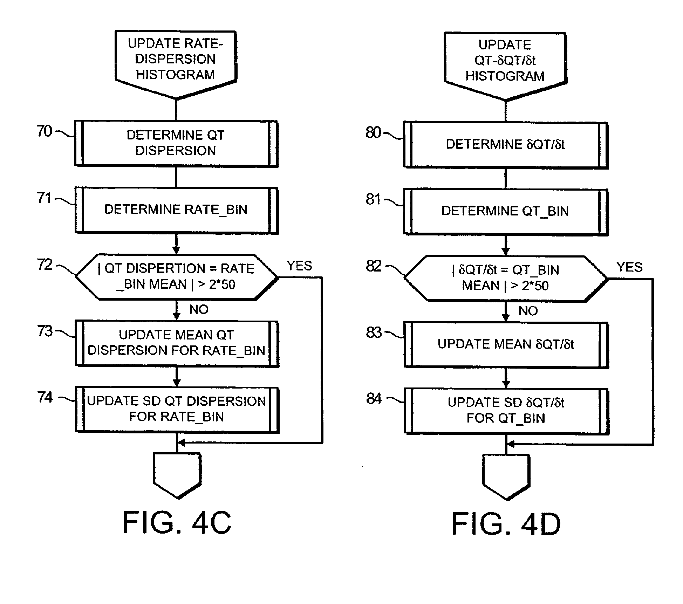

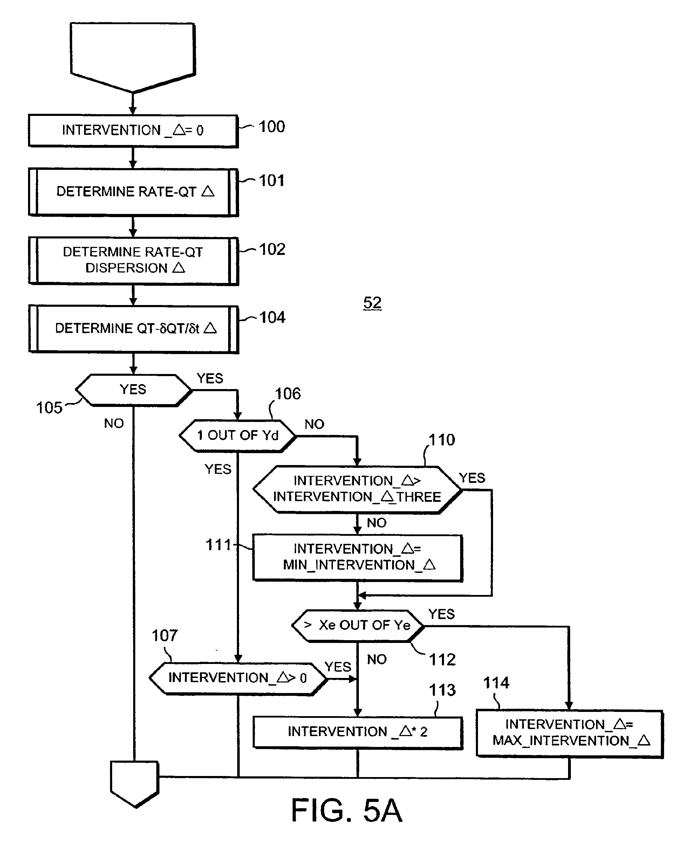

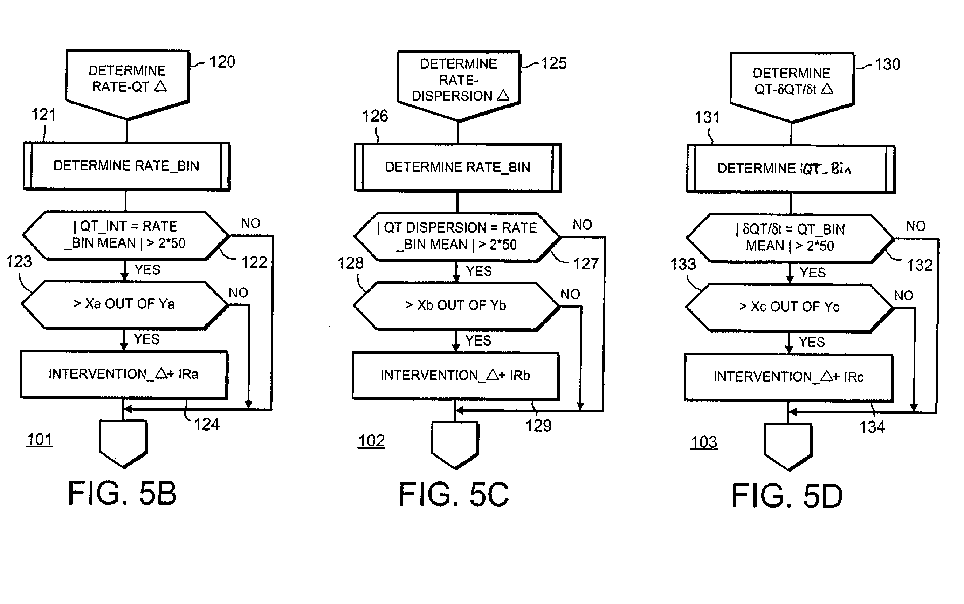

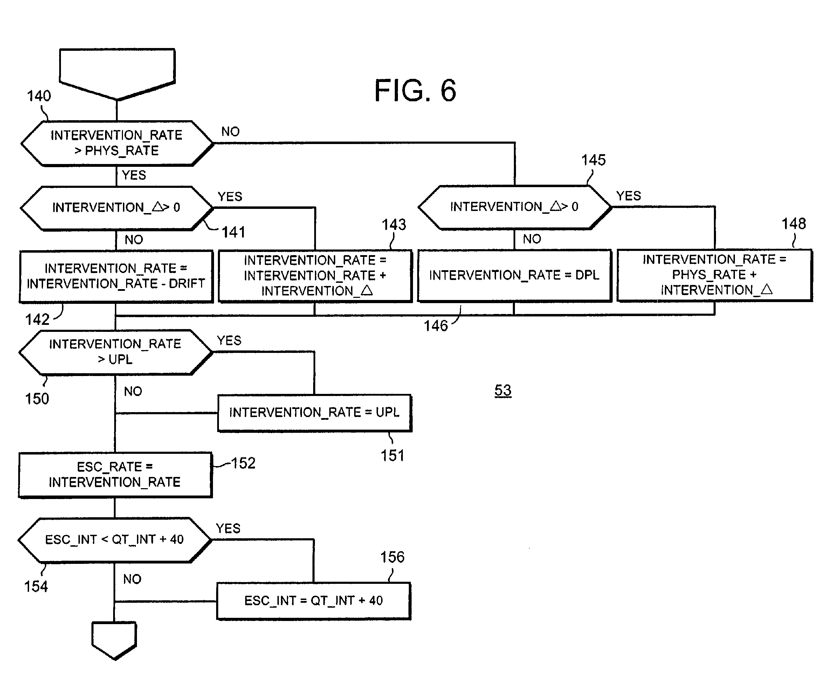

Figures 2 to 6 relate to features of embodiments not described in this specification.
Figure 7 is an overview flow diagram illustrating the cyclical operations of the VT Prevention routine in accordance with this invention.
Figure 8 is a simplified flow diagram illustrating the primary steps taken in carrying out the Learning Phase portion of the VT Prevention routine.
Figure 9 is a simplified flow diagram illustrating the primary steps taken in carrying out the Ventricular Extra Systole analysis portion of the VT Prevention Routine.
Figure 10 is a simplified flow diagram illustrating the primary steps taken in carrying out the Determine Intervention portion of the VT Prevention routine.

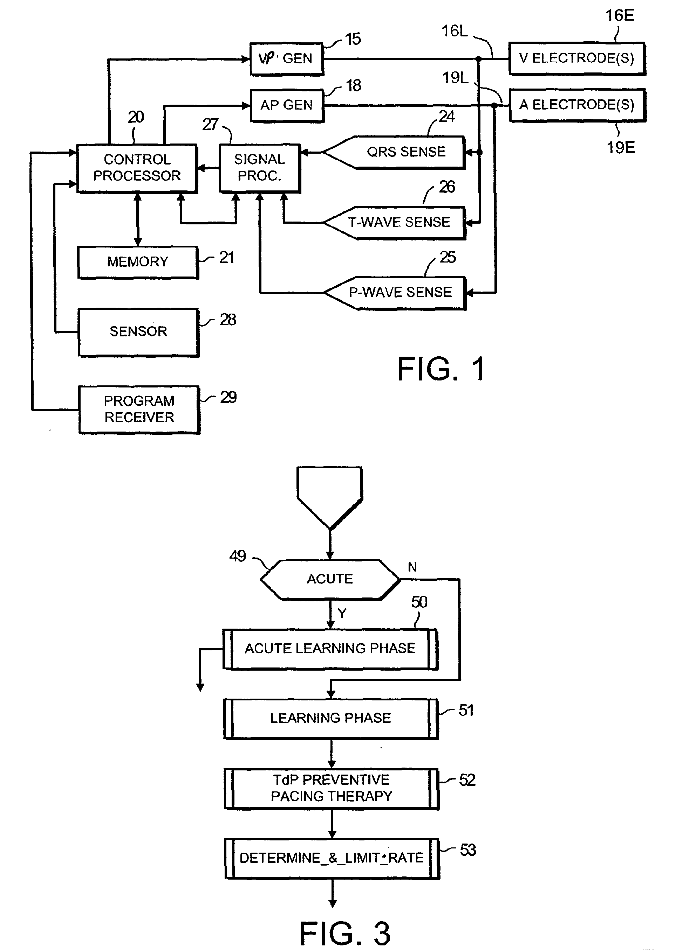

Referring now to Figure 1, there is shown a block diagram of the primary functional components of an illustrative pacemaker system for use in this invention. A VP generator 15 provides pacing pulses, generated under control of block 20, for delivery through lead 16 to one or more ventricular electrodes 16E located in the patient's right ventricle. Likewise, AP generator 18 provides atrial pacing pulses, also generated under control of block 20, for delivery through lead 19 to one or more atrial electrodes 19E located in the patient's right atrium. While not shown, it is understood that the invention is equally applicable to single chamber and to other multi-chamber configurations. Signals sensed by electrodes 16E are connected to QRS Sense circuit 24 which amplifies, the signals and provides V-Sense, or VS signals to signal processing block 27. Signals from ventricular electrodes 16E are also passed to T-Wave sense circuit 26, which provides T-Sense signals to block 27. Signals from atrial electrodes 19E are connected to P-Wave sense circuit 25, which outputs A-Sense, or AS signals to block 27.

Block 27 suitably contains dedicated signal processing hardware; and includes an A-D converter for converting the signals into digital form. The QRS and T-wave template generation and comparison steps, referred to below, are performed wholly or in part in this circuitry. The digital signals from block 27 are transferred to block 20 for further processing and/or storage. Block 20 controls the pacemaker functions, e.g., the cyclical functions of setting and timing out escape intervals; receiving sensed signals from the patient's heart and resetting escape intervals based on those signals; and carrying out special functions such as the VT Prevention function of this invention. Block 20 preferably comprises a microprocessor and associated memory, shown at 21, for storing the required software routines.

The memory 21 suitably includes dedicated RAM and ROM. Control parameters and values can be programmed from an external programmer through program receiver 29, in a known manner. The pacemaker can be programmed to operate in different modes. Sensor 28 may be used to provide a rate responsive parameter, e.g. activity, to be used alone or in combination with another parameter such as QT, in a manner known in the art.

The following describes the place of the VT Prevention routine within the overall main flow which is carried out cyclically. The main flow is entered cyclically and the pacemaker performs various bookkeeping and other steps. The spontaneous rate is determined; for a dual chamber system, decision rates are updated, in a manner as disclosed in U.S. Patent No. 5,247,930, assigned to the assignee of this invention. Then, the pacemaker carries out a routine for determining the patient's circadian pattern, in order to determine "daytime" and "nighttime" for the patient, and to determine the patient's awakening period. Then, the VT Prevention routine of this invention is performed, as discussed in detail below. If the result of this routine is the setting of an intervention rate in response to a determination of onset of VT, the intervention rate controls the setting of the ventricular escape interval. These atrial and ventricular escape intervals are set and then the pacemaker carries out the rest of the main flow, including ventricular event handling. QRS and T-wave sensing is also done.

Reference is made to U.S. patent application S.N. 08/800,413, filed February 14, 1997, "Pacemaker With Automatic Lower Rate Limit Drop". The referenced disclosure sets forth several embodiments of pacemaker routines for determining when the patient enters nighttime and when the patient enters daytime, by monitoring and evaluating changes in QT interval. Transition from nighttime to daytime is shown as representing the start of the awakening period. The awakening period may suitably be determined in several ways. For example, the awakening period may be determined as extending from the nighttime/daytime transition for a period of, e.g., one hour. In this arrangement, the end of nighttime is suitably determined as the time when the QT interval drops below the variable QT_sleep. Alternatively, in another embodiment, nighttime may be of a fixed duration, with the start of night being determined when the QT interval stays above the QT_sleep variable for a night_criterion duration. In this case, the awakening period may be set so as to straddle the nighttime/daytime transition, e.g., from half an hour before to half an hour after. It is noted that other embodiments may be used for determining the end of night, e.g., by determining when the activity rate rises above a nighttime level.

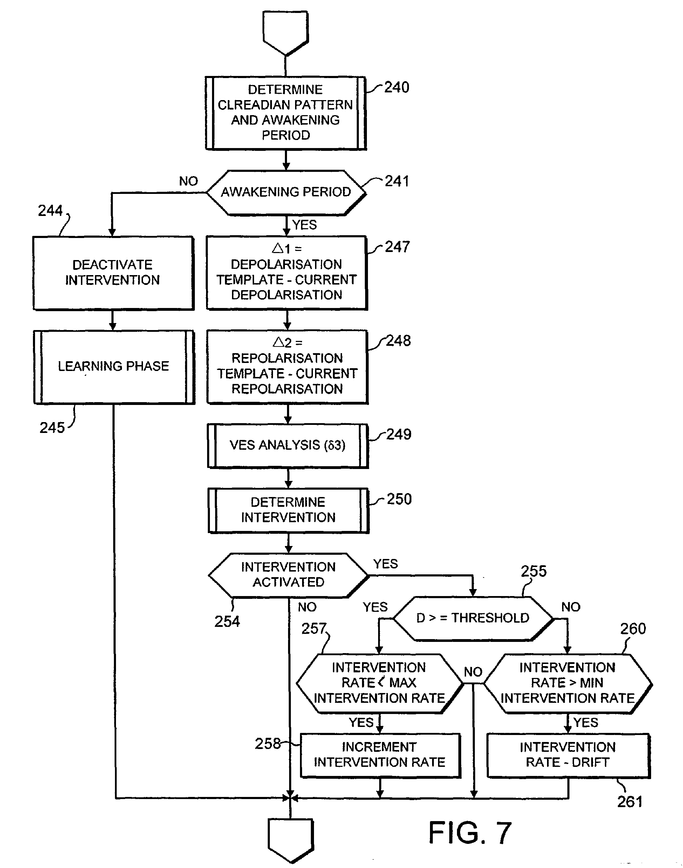

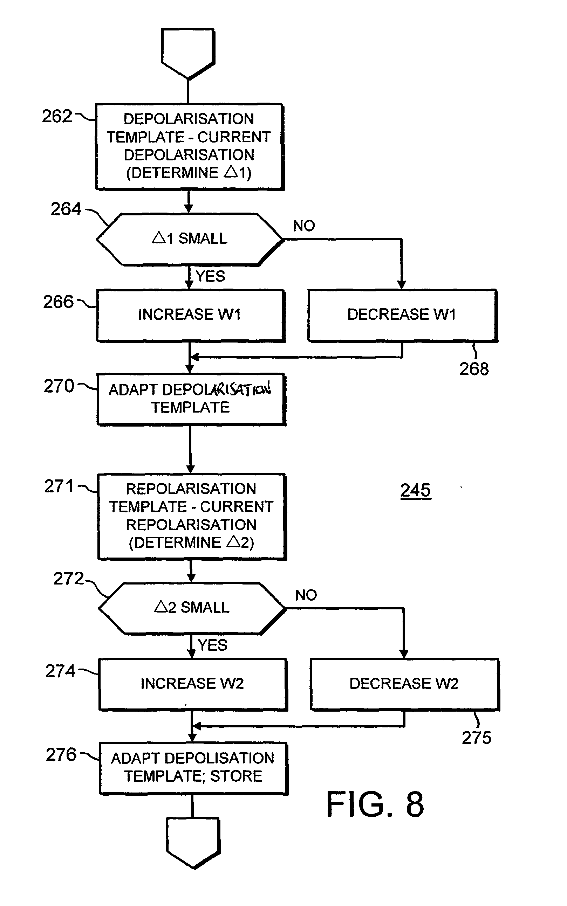

Referring now to Figure 7, there is shown a flow diagram of the specific steps taken in accordance with the VT prevention feature of this invention. At 240, the routine is run for determining the awakening period. At 241 the pacemaker determines whether the patient is in fact in the awakening period. If no, at 244 the intervention state is deactivated, and at 245 the pacemaker goes into the learning phase, the details of which are set forth in connection with Figure 8.

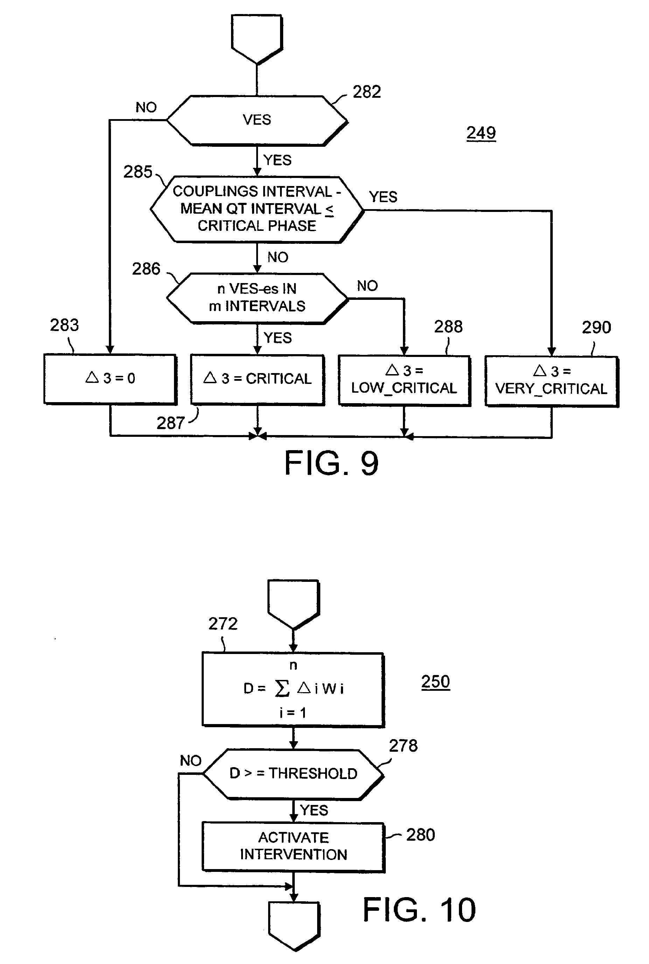

If the patient is in the awakening period, the pacemaker goes on to process current information concerning the depolarization (QRS) and repolarization (T wave) waveforms. At 247, the pacemaker obtains the depolarization template for the current cycle, and compares it to the depolarization template which was generated during the learning phase. The difference is computed and stored as Δ1. Likewise, at step 248, the pacemaker gets the current repolarization template and compares it with the stored repolarization template from the learning phase, and generates a Δ2, which is representative of the difference. Then, at block 249, the pacemaker goes through a VES analysis, to obtain a measure (Δ3) of whether there has been a ventricular extra systole, and how close the coupling interval was to the patient's mean QT interval. The VES analysis is set forth in particular detail in Figure 9. Following this, the pacemaker goes to block 250, and determines whether intervention is indicated, based upon data gathered and generated at blocks 247, 248, and 249 above. The Determine Intervention routine is set forth in detail at Figure 10.

Still referring to Figure 7, at block 254, the pacemaker determines whether intervention has been activated. If no, the routine exits, and the pacemaker continues to set the pacing escape intervals in a normal way. However, if yes, the routine branches to block 255, and determines whether the variable D is equal to or greater than a predetermined threshold. D is calculated in the Determine Intervention routine 250, and represents a summation of the respective Δ values calculated at blocks 247, 248, which are representative of refractorines dispersion; and also the Δ value calculated at 249, which represents the presence of a dangerous VES. The calculation of D is discussed in more detail in connection with Figure 10. If D is equal to or greater than threshold, at 57 the pacemaker determines whether the intervention rate remains less than the maximum intervention rate. If yes, intervention rate is incremented at 258; if no, intervention rate is at its maximum allowable value and the routine exits. If, at 255, D is not up to threshold, then at 260 it is determined whether the intervention rate is higher than the minimum intervention rate. If yes, at 261 intervention rate is decremented by subtracting a programmable drift value; if no, the intervention rate is as low as is allowed, and the routine exits.

Referring now to Figure 8, there is shown a flow diagram of the learning phase routine 245, in accordance with this invention. As discussed in connection with Figure 7, this phase is entered when the patient is not in the awakening period. At 262, the pacemaker calculates a variable Δ1 which constitutes the depolarization template then stored in memory, minus the current depolarization template for the just detected R wave. In obtaining a template, the wave signal data is placed into digital form by an A-D converter, which is part of the function provided by signal processor block 27. Obtaining waveform templates is well known in the art, and any suitable hardware or software arrangement can be used in this invention. In a preferred embodiment, digital samples are obtained representing the waveform amplitude along successive time increments, from the beginning of the wave to the end, and are stored. In determining the Δ difference, the respective waveform amplitude values are subtracted, and the difference is integrated over the time domain. In a preferred embodiment, the template generation and template difference calculations are performed by dedicated hardware, as shown at 27. However, any combination of hardware and software can be utilized.

Next, at 264, it is determined whether the Δ1 value is small. The reason for making this determination is that large variations during the learning phase suggest that the signal is not stable enough to be a reference during the awakening period. Consequently, if the deviation found at 264 is statistically small (indicating stability), at 266 a weighting factor W1 is increased; if the deviation is not statistically small, then at 268 the W1 is decreased. These weighting factors are utilized in the subsequent determination of intervention in routine 250. Next, at block 270, the depolarization template is adapted so as to be changed toward the most recently detected depolarization template. This can be done, e.g., by matching the minimum values and slopes of the depolarization template and the new QRS wave, and adjusting each sample of the template incrementally toward the samples of the new QRS. The functions are suitably carried out by the microprocessor of block 20.

Still referring to Figure 8, blocks 271, 272 and 274-276 represent corresponding steps for the repolarization template, which reflects the sensed T wave. At 271, the deviation Δ2 is determined, by subtracting the just obtained repolarization wave from the stored repolarization template. At 272, it is determined whether the deviation is small, representing a stable signal. If yes, weighting factor W2 is increased at 274; if no, W2 is decreased at 275. The repolarization template is then adapted and stored at 276.

Referring now to Figure 9, there is shown a flow diagram of the routine 249 for carrying out VES analysis. This is done, as discussed above, because VT is often initiated or preceded by one or more ventricular extra-systoles if the patient heart rate is low enough and the coupling interval is critical, i.e., VES occurs near or in the vulnerable phase. In this situation likewise, intervention may be indicated, so the pacemaker of this invention collects VES data which is included in the Determine Intervention routine.

At block 282, it is determined whether there has been a VES. If no, the routine branches to block 283, and sets Δ3 (the deviation value corresponding to VES analysis) to zero. However, if there has been a VES, then it has to be determined how critical the VES is deemed, i.e., the deviation needs to be weighted. At 285, the pacemaker compares the coupling interval (the interval from the prior R wave to the VES) to the mean QT interval as stored. If the coupling interval minus the mean QT interval is less than or equal to a stored critical phase value, then the VES is deemed very critical, and at 290 Δ3 is given a weighted VERY_CRITICAL value. However, if the answer at 285 is no, then the routine goes to 286 and determines whether there have been a predetermined number n VES occurrences in the last m intervals, where n and m are programmable numbers. If yes, the VES occurrence is deemed critical, and at block 287 Δ3 is given a weighted CRITICAL value which is somewhat less than the VERY_CRITICAL value. If the answer at 286 is no, at 288 Δ3 is given a LOW_CRITICAL weighting. At routine 249, the values assigned to Δ3 include the weighting factor, such that the stored deviation is assumed to be accompanied by a weighting factor of 1 for the calculation which is carried out at block 277.

Referring now to Figure 10, there is shown a flow diagram of the Determined Intervention routine 250. At step 277, the pacemaker determines the total deviation D, which is calculated by taking the sum of all the separate deviations, each multiplied by its respective weighting factor W. In the preferred embodiment as illustrated, there are three different deviations determined, so the summation is from i = 1 to i = 3. Thus, each cycle the summation constitutes Δ1 multiplied by the current value of W1; Δ2 multiplied by the current value of W2, and the determined value of .3, where the weighting factor is 1 since the value of Δ3 has already been calculated to reflect appropriate weighting. At 278, it is determined whether the current value D is greater than or equal to threshold. If no, intervention is not indicated and the routine exits. However, if D is greater than or equal to the programmed threshold, then intervention is activated at step 280, suitably by setting a flag to store the fact that intervention has been activated. As seen in Figure 7, once intervention has been activated, it is not deactivated until the awakening period is over, at which time the pacemaker proceeds to block 244 and deactivates intervention. As per the above discussion of Figure 7, if D drops below threshold when Intervention is activated, the Intervention rate is decremented toward a lower limit.

Recapitulating, and referring to Figure 7, it is seen that during normal periods outside of the awakening period, both daytime and nighttime, the pacemaker is continually adapting the depolarization and repolarization templates in the learning phase. When the patient is in the awakening period, data represented by deviation values Δ1, Δ2 and Δ3 are obtained at blocks 247, 248 and 249 respectively. Whenever, during the awakening period, the cumulative sum of the deviations exceeds a predetermined threshold, intervention is activated, and the intervention mode is maintained throughout the awakening period. Of course, if the deviation values, which represent refractory dispersion, become small, then the intervention rate drifts down to a lower rate limit, such that there effectively is no overdrive intervention. However, as long as the wave variability remains high, the intervention rate will be maintained so as to provide overdrive pacing calculated to prevent ventricular tachycardia.

It is noted that while three separate deviation measurements are illustrated in the preferred embodiments, additional data can be collected, and weighted accordingly. Thus, each cycle a direct measure of QTc or QT_{c} can be obtained and a value of QT dispersion calculated and weighted; this weighted QT dispersion value is then added to the calculation made for determining intervention. Additionally, each weighting factor can be programmed to vary within predetermined limits, so that weighting can be adapted in terms of known patient history.

## Claims

1. A pacemaker system for pacing a patient's heart, having ventricular pacing (VP) means for generating and delivering pacing pulses to a patient's ventricle, rate control means for controlling the rate of said pacing, pulses, and said rate control means having ventricular tachycardia (VT) prevention means for controlling said rate to an intervention rate when said patient has a detected measure of refractory dispersion during awakening, said VT prevention means comprising dispersion means for determining a measure of dispersion of ventricular refractoriness; **characterized in that** said VT prevention means further comprises
awakening means for determining when said patient is in a state of awakening;
and
intervention means for setting and continually adjusting said intervention rate as a function of said dispersion measure.

2. The system as described in claim 1, wherein said dispersion means comprises means for cyclically obtaining a measure of the variability of the patient's QRS during the awakening state.

3. The system as described in claim 1, wherein said dispersion means comprises means for obtaining a reference measure of the patient's QRS waveform when the patient is not awakening, means for obtaining a current measure of the patient's current QRS waveform during awakening, and comparing means for comparing said currents measure with said reference measure.

4. The system as described in claim 1, wherein said dispersion means comprises means for cyclically obtaining a measure of the variability of the patient's T wave during the awakening state.

5. The system as described in claim 1, wherein said dispersion means comprises means for obtaining a reference measure of the patient's T wave when the patient is not awakening, means for obtaining a current measure of the patient's current T wave during awakening, and comparing means for comparing said current measure with said reference measure.

6. The system as described in claim 1, wherein said dispersion means comprises QT means for cyclically obtaining a measure of the variability of the patient's QT interval during the awakening state.

7. The system as described in any of claims 1 to 6, further comprising VES means for detecting when a ventricular extra systole occurs during the awakening state that is close to the end of the ventricular refractory period, and wherein said intervention means comprises determining means for determining said intervention rate as a function of said VES detecting.

8. The system as described in claim 1, wherein said dispersion means comprises means for obtaining at least two respective measures of said dispersion, and accumulating means for accumulating said measures, and wherein said intervention means adjusts said intervention rate as a function of said accumulated measures.

9. The system as described in any of claims 1 to 8, comprising rate means for determining the patient's spontaneous rate, and wherein said VT prevention means comprises means for setting said intervention right higher than said spontaneous rate, whereby said pacemaker overdrives the patient's spontaneous rate during said awakening state.

10. The system as described in claim 9, wherein said intervention means comprises increment means for incrementing said intervention rate when said accumulated measures increase, and for decrementing said intervention rate when said accumulated measures decrease.

11. The system as described in claim 1, wherein said dispersion means comprises data means for cyclically obtaining data representative of ventricular refractoriness.

12. A system as claimed in claim 1 further comprising ventricular sensing (VS) means for sensing ventricular QRS and T waves and rate means for determining the patient's spontaneous heart rate, wherein said rate control means are adapted for controlling when said VP means generates and delivers ventricular pacing pulses, wherein said dispersion means comprises:
depolarization means for determining a first measure of dispersion of the patient's depolarization wave during the awakening period;
repolarization means for determining a second measure of dispersion of the patient's repolarization wave during the awakening period.
and wherein said dispersion means are further adapted for controlling said VP means to generate and deliver pacing pulses at an intervention rate that overrides the patient's spontaneous rate as a function of said first and second measures.

13. The system as described in claim 12, wherein said VT prevention means comprises learning means operative during the patient's non-awakening period for generating and storing reference signals representative of the patient's depolarization and repolarization waves during said non-awakening period, and current means for obtaining current signals representative of said depolarization and repolarization signals during said awakening period.

14. The system as described in claim 13, wherein said learning means comprises template means for obtaining and storing reference depolarization and repolarization templates, and said current means comprises current template means for cyclically obtaining and storing current depolarization and repolarization templates.

15. The system as described in claim 14, wherein said depolarization means comprises first comparison means for comparing the current depolarization template with said reference depolarization template to obtain a depolarization deviation value, and said repolarization means comprises second comparison means for comparing the current repolarization template with said reference repolarization template to obtain a repolarization deviation value.

16. The system as described in claim 15, wherein said depolarization means comprises first weighting means for weighting said depolarization deviation value as a function of depolarization wave stability to obtain said first measure, and said repolarization means comprises second weighting means for weighting said repolarization deviation value as a function of repolarization wave stability to obtain said second measure.

17. The system as described in any of claims 12 to 16, wherein said VT prevention means further comprises means operative during said awakening period for accumulating weighted data representative of depolarization dispersion and repolarization dispersion.

18. A system as claimed in claim 1, wherein said dispersion means comprises
dispersion data means for determining during said awakening period data representative of ventricular refractory dispersion; and further comprising
threshold means for determining from said data when patient refractory dispersion exceeds said threshold; and wherein said intervention means comprises overdrive control means for setting the pacing rate at an intervention rate which is adjusted as a function of said data.

19. The system as described in claim 18, wherein said dispersion data means comprises first means for obtaining data representative of a first predetermined parameter of the QRS-T wave signals and second means for obtaining data representative of a second predetermined parameter of the QRS-T wave signals.

## Patentansprüche

1. Herzschrittmachersystem zum Schrittmachen bzw. Vorgeben des Rhythmus eines Patientenherzens, mit Kammerschrittmachermitteln (VP-Mitteln) zum Erzeugen und Ausgeben von Schrittmacherimpulsen an eine Patientenkammer und Frequenz- bzw. Ratensteuermitteln zum Steuern der Frequenz bzw. der Rate der Schrittmacherimpulse, wobei die Ratensteuermittel Kammertachykardie-Verhinderungsmittel (VT-Verhinderungsmittel) besitzen, um die Rate auf eine Interventionsrate zu regeln bzw. zu steuern, wenn der Patient beim Aufwachen ein erfasstes Maß an refraktärer Dispersion aufweist, wobei die VT-Verhinderungsmittel Dispersionsmittel aufweisen, um ein Maß der Dispersion der Kammerrefraktärität zu bestimmen; **dadurch gekennzeichnet, dass** die VT-Verhinderungsmittel ferner umfassen:
Aufwachmittel zum Bestimmen, wann ein Patient in einem Aufwachzustand ist; und
Interventionsmittel zum Festlegen und kontinuierlichen Einstellen der Interventionsrate als Funktion des Dispersionsmaßes.

2. System nach Anspruch 1, bei dem die Dispersionsmittel Mittel zum zyklischen Erhalten eines Maßes der Variabilität der Patienten-QRS während des Aufwachzustandes aufweisen.

3. System nach Anspruch 1, bei dem die Dispersionsmittel versehen sind mit Mitteln zum Erhalten eines Referenzmaßes der Patienten-QRS-Signalform, wenn der Patient nicht aufwacht, mit Mitteln zum Erhalten eines momentanen Maßes der momentanen Patienten-QRS-Signalform während des Aufwachens und mit Vergleichsmitteln zum Vergleichen des momentanen Maßes mit dem Referenzmaß.

4. System nach Anspruch 1, bei dem die Dispersionsmittel versehen sind mit Mitteln zum zyklischen Erhalten eines Maßes der Variabilität der Patienten-T-Welle während des Aufwachzustandes.

5. System nach Anspruch 1, bei dem die Dispersionsmittel versehen sind mit Mitteln zum Erhalten eines Referenzmaßes der Patienen-T-Welle, wenn der Patient nicht aufwacht, mit Mitteln zum Erhalten eines momentanen Maßes der momentanen Patienten-T-Welle während des Aufwachens und mit Vergleichsmitteln zum Vergleichen des momentanen Maßes mit dem Referenzmaß.

6. System nach Anspruch 1, bei dem die Dispersionsmittel versehen sind mit QT-Mitteln zum zyklischen Erhalten eines Maßes der Variabilität des Patienten-QT-Intervalls während des Aufwachzustandes.

7. System nach einem der Ansprüche 1 bis 6, das ferner VES-Mittel zum Feststellen, wann eine Kammer-Extrasystole während des Aufwachzustandes in der Nähe des Endes der Kammer-Refraktärperiode auftritt, aufweist, wobei die Interventionsmittel Bestimmungsmittel zum Bestimmen der Interventionsrate als Funktion der VES-Erfassung aufweisen.

8. System nach Anspruch 1, bei dem die Dispersionsmittel versehen sind mit Mitteln zum Erhalten von wenigstens zwei Dispersionsmaßen, und mit Akkumulationsmitteln zum Akkumulieren dieser Maße, wobei die Interventionsmittel die Interventionsrate als Funktion der akkumulierten Maße einstellen.

9. System nach einem der Ansprüche 1 bis 8, das versehen ist mit Ratenmitteln zum Bestimmen der spontanen Patienten-Rate, wobei die VT-Verhinderungsmittel Mittel zum Einstellen der Interventionsrate höher als die spontane Rate aufweisen, wodurch der Herzschrittmacher die spontane Patienten-Rate während des Aufwachzustandes übersteuert.

10. System nach Anspruch 9, bei dem die Interventionsmittel Inkrementierungsmittel zum Inkrementieren der Interventionsrate, wenn die akkumulierten Maße zunehmen, und zum Dekrementieren der Interventionsrate, wenn die akkumulierten Maße abnehmen, aufweisen.

11. System nach Anspruch 1, bei dem die Dispersionsmittel Datenmittel zum zyklischen Erhalten von Daten, die die Kammerrefraktärität darstellen, aufweisen.

12. System nach Anspruch 1, das ferner versehen ist mit Kammererfassungsmitteln (VS-Mittel) zum Erfassen einer Kammer-QRS und von T-Wellen und mit Ratenmitteln zum Bestimmen der spontanen Patienten-Herzrate bzw.
- frequenz, wobei die Raten- bzw. Frequenzsteuermittel für eine Regelung bzw. Steuerung ausgelegt sind, wenn die VP-Mittel Kammer-Schrittmacherimpulse erzeugen und ausgeben,
wobei die Dispersionsmittel aufweisen:
Depolarisationsmittel zum Bestimmen eines ersten Dispersionsmaßes der Patienten-Depolarisationswelle während der Aufwachperiode;
Neupolarisationsmittel zum Bestimmen eines zweiten Dispersionsmaßes der Patienten-Neupolarisationswelle während der Aufwachperiode,
und wobei die Dispersionsmittel ferner so beschaffen sind, dass sie die VP-Mittel steuern, damit sie Schrittmacherimpulse mit einer Interventionsrate, die die spontane Patienten-Rate übersteuert, als Funktion des ersten und des zweiten Maßes erzeugen und ausgeben.

13. System nach Anspruch 12, bei dem die VT-Verhinderungsmittel versehen sind mit Lernmitteln, die während der Nichtaufwachperiode des Patienten arbeiten können, um Referenzsignale zu erzeugen und zu speichern, die die Depolarisations- und Neupolarisationswellen des Patienten während der Nichtaufwachperiode darstellen, und mit Momentanmitteln zum Erhalten momentaner Signale, die die Depolarisations- und Neupolarisationssignale während der Aufwachperiode darstellen.

14. System nach Anspruch 13, bei dem die Lernmittel Schablonenmittel zum Erhalten und Speichern von Referenz-Depolarisations- und Referenz-Neupolarisationsschablonen aufweisen und die Momentanmittel Momentanschablonenmittel zum zyklischen Erhalten und Speichern der momentanen Depolarisations- und Neupolarisationsschablonen aufweisen.

15. System nach Anspruch 14, bei dem die Depolarisationsmittel erste Vergleichsmittel zum Vergleichen der momentanen Depolarisationsschablone mit der Referenz-Depolarisationsschablone, um einen Depolarisationsabweichungswert zu erhalten, aufweisen und die Neupolarisationsmittel zweite Vergleichsmittel zum Vergleichen der momentanen Neupolarisationsschablone mit der Referenz-Neupolarisationsschablone, um einen Neupolarisationsabweichungswert zu erhalten, aufweisen.

16. System nach Anspruch 15, bei dem die Depolarisationsmittel erste Gewichtungsmittel zum Gewichten des Depolarisationsabweichungswertes als Funktion der Depolarisationswellen-Stabilität, um das erste Maß zu erhalten, aufweisen und die Neupolarisationsmittel zweite Gewichtungsmittel zum Gewichten des Neupolarisationsabweichungswertes als Funktion der Neupolarisationswellen-Stabilität, um das zweite Maß zu erhalten, aufweisen.

17. System nach einem der Ansprüche 12 bis 16, bei dem die VT-Verhinderungsmittel ferner Mittel aufweisen, die während der Aufwachperiode arbeiten können, um gewichtete Daten zu akkumulieren, die die Depolarisationsdispersion und die Neupolarisationsdispersion darstellen.

18. System nach Anspruch 1, bei dem die Dispersionsmittel aufweisen:
Dispersionsdatenmittel zum Bestimmen von Daten während der Aufwachperiode, die eine Kammer-Refraktärdispersion darstellen; und ferner aufweisen:
Schwellenwertmittel zum Bestimmen aus den Daten, wann die Refraktärdispersion des Patienten den Schwellenwert übersteigt;
wobei die Interventionsmittel Übersteuerungsmittel zum Setzen der Schrittmacherrate auf eine Interventionsrate, die als Funktion dieser Daten eingestellt wird, aufweisen.

19. System nach Anspruch 18, bei dem die Dispersionsdatenmittel versehen sind mit ersten Mitteln zum Erhalten von Daten, die einen ersten vorgegebenen Parameter der QRS-T-Wellensignale darstellen, und mit zweiten Mitteln zum Erhalten von Daten, die einen zweiten vorgegebenen Parameter der QRS-T-Wellensignale darstellen.

## Revendications

1. Système de stimulateur cardiaque pour stimuler le coeur d'un patient, ayant des moyens de stimulation ventriculaire (VP) pour générer et délivrer des impulsions de stimulation à un ventricule du patient, des moyens de commande de débit pour commander le débit desdites impulsions de stimulation, et lesdits moyens de commande de débit ayant des moyens de prévention de tachycardie ventriculaire (VT) pour commander ledit débit à un débit d'intervention lorsque ledit patient a une mesure de dispersion de réfractaire détectée durant l'éveil, lesdits moyens de prévention de VT comportant des moyens de détermination de dispersion pour déterminer une mesure de dispersion de réfractarité ventriculaire, **caractérisé en ce que** lesdits moyens de prévention de VT comportent de plus :
des moyens de détermination d'éveil pour déterminer lorsque ledit patient est dans un état d'éveil, et
des moyens d'intervention pour régler et continuellement ajuster ledit débit d'intervention en fonction de ladite mesure de dispersion.

2. Système selon la revendication 1, dans lequel lesdits moyens de détermination de dispersion comportent des moyens pour obtenir de manière cyclique une mesure de la variabilité de la forme d'onde QRS du patient durant l'état d'éveil.

3. Système selon la revendication 1, dans lequel lesdits moyens de détermination de dispersion comportent des moyens pour obtenir une mesure de référence de la forme d'onde QRS du patient lorsque le patient n'est pas en éveil, des moyens pour obtenir une mesure courante de la forme d'onde QRS courante du patient durant l'éveil, et des moyens de comparaison pour comparer ladite mesure courante à ladite mesure de référence.

4. Système selon la revendication 1, dans lequel lesdits moyens de détermination de dispersion comportent des moyens pour obtenir de manière cyclique une mesure de la variabilité de l'onde T du patient durant l'état d'éveil.

5. Système selon la revendication 1, dans lequel lesdits moyens de détermination de dispersion comportent des moyens pour obtenir une mesure de référence de l'onde T du patient lorsque le patient n'est pas en éveil, des moyens pour obtenir une mesure courante de l'onde T courante du patient durant l'éveil, et des moyens de comparaison pour comparer ladite mesure courante à ladite mesure de référence.

6. Système selon la revendication 1, dans lequel lesdits moyens de détermination de dispersion comportent des moyens d'indication de QT pour obtenir de manière cyclique une mesure de la variabilité de l'intervalle QT du patient durant l'état d'éveil.

7. Système selon l'une quelconque des revendications 1 à 6, comportant de plus des moyens de détection de VES pour détecter lorsqu'une extrasystole ventriculaire se produit durant l'état d'éveil qui est proche de la fin de la période réfractaire ventriculaire, et dans lequel lesdits moyens d'intervention comportent des moyens de détermination pour déterminer ledit débit d'intervention en fonction de ladite détection de VES.

8. Système selon la revendication 1, dans lequel lesdits moyens de détermination de dispersion comportent des moyens pour obtenir au moins deux mesures respectives de ladite dispersion, et des moyens d'accumulation pour cumuler lesdites mesures, et dans lequel lesdits moyens d'intervention ajustent ledit débit d'intervention en fonction desdites mesures cumulées.

9. Système selon l'une quelconque des revendications 1 à 8, comportant des moyens de détermination de débit pour déterminer le débit spontané du patient, et dans lequel lesdits moyens de prévention de VT comportent des moyens pour régler ledit débit d'intervention supérieur audit débit spontané, de manière à ce que ledit stimulateur cardiaque surmultiplie le débit spontané du patient durant l'état d'éveil.

10. Système selon la revendication 9, dans lequel lesdits moyens d'intervention comportent des moyens d'incrémentation pour incrémenter ledit débit d'intervention lorsque lesdites mesures cumulées augmentent, et pour décrémenter ledit débit d'intervention lorsque lesdites mesures cumulées décroissent.

11. Système selon la revendication 1, dans lequel lesdits moyens de détermination de dispersion comportent des moyens d'obtention de données pour obtenir de manière cyclique des données représentatives d'une réfractarité ventriculaire.

12. Système selon la revendication 1, comportant de plus des moyens de détection ventriculaire (VS) pour détecter des ondes QRS et T ventriculaires et des moyens de détermination de débit pour déterminer le débit cardiaque spontané du patient, dans lequel lesdits moyens de commande de débit sont adaptés pour commander lorsque lesdits moyens de stimulation VP génèrent et délivrent des impulsions de stimulation ventriculaire, dans lequel lesdits moyens de détermination de dispersion comportent :
des moyens de dépolarisation pour déterminer une première mesure de dispersion de l'onde de dépolarisation du patient durant la période d'éveil,
des moyens de repolarisation pour déterminer une seconde mesure de dispersion de l'onde de repolarisation du patient durant la période d'éveil,
et dans lequel lesdits moyens de détermination de dispersion sont de plus adaptés pour commander lesdits moyens de stimulation VP pour générer et délivrer des impulsions de stimulation à un débit d'intervention qui surmultiplie le débit spontané du patient en fonction desdites première et seconde mesures.

13. Système selon la revendication 12, dans lequel lesdits moyens de prévention de VT comportent des moyens d'apprentissage opérationnels durant la période de non-éveil du patient pour générer et mémoriser des signaux de référence représentatifs des ondes de dépolarisation et de repolarisation du patient durant ladite période de non-éveil, et des moyens de mesure courante pour obtenir des signaux courants représentatifs desdits signaux de dépolarisation et de repolarisation durant ladite période d'éveil.

14. Système selon la revendication 13, dans lequel lesdits moyens d'apprentissage comportent des moyens d'obtention de modèle pour obtenir et mémoriser des modèles de dépolarisation et de repolarisation de référence, et lesdits moyens de mesure courante comportent des moyens d'obtention de modèle courant pour obtenir et mémoriser de manière cyclique des modèles de dépolarisation et de repolarisation courants.

15. Système selon la revendication 14, dans lequel lesdits moyens de dépolarisation comportent des premiers moyens de comparaison pour comparer le modèle de dépolarisation courant audit modèle de dépolarisation de référence pour obtenir une valeur d'écart de dépolarisation, et lesdits moyens de repolarisation comportent des seconds moyens de comparaison pour comparer le modèle de repolarisation courant audit modèle de repolarisation de référence pour obtenir une valeur d'écart de repolarisation.

16. Système selon la revendication 15, dans lequel lesdits moyens de dépolarisation comportent des premiers moyens de pondération pour pondérer ladite valeur d'écart de dépolarisation en fonction de la stabilité d'onde de dépolarisation pour obtenir ladite première mesure, et lesdits moyens de repolarisation comportent des seconds moyens de pondération pour pondérer ladite valeur d'écart de repolarisation en fonction de la stabilité d'onde de repolarisation pour obtenir ladite seconde mesure.

17. Système selon l'une quelconque des revendications 12 à 16, dans lequel lesdits moyens de prévention de VT comportent de plus des moyens opérationnels durant ladite période d'éveil pour cumuler des données pondérées représentatives d'une dispersion de dépolarisation et d'une dispersion de repolarisation.

18. Système selon la revendication 1, dans lequel lesdits moyens de détermination de dispersion comportent :
des moyens de détermination de données de dispersion pour déterminer durant ladite période d'éveil des données représentatives d'une dispersion de réfractaire ventriculaire, et comportant de plus :
des moyens de détermination de seuil pour déterminer d'après lesdites données lorsqu'une dispersion de réfractaire du patient dépasse ledit seuil,
et dans lequel lesdits moyens d'intervention comportent des moyens de commande de surmultiplication pour régler le débit de stimulation à un débit d'intervention qui est ajusté en fonction desdites données.

19. Système selon la revendication 18, dans lequel lesdits moyens de détermination de données de dispersion comportent des premiers moyens pour obtenir des données représentatives d'un premier paramètre prédéterminé des signaux d'onde QRS-T et des seconds moyens pour obtenir des données représentatives d'un second paramètre prédéterminé des signaux d'onde QRS-T.
